(19) **Europäisches Patentamt**

**European Patent Office**

**Office européen des brevets**

(11) **EP 1 098 657 B1**

(12) **FASCICULE DE BREVET EUROPEEN**

(45) Date de publication et mention
de la délivrance du brevet:
**10.07.2002 Bulletin 2002/28**

(51) Int Cl.⁷: **A61K 35/78, A61P 3/04**

(86) Numéro de dépôt international:
**PCT/FR00/00065**

(21) Numéro de dépôt: **00900573.7**

(22) Date de dépôt: **14.01.2000**

(87) Numéro de publication internationale:
**WO 00/41708 (20.07.2000 Gazette 2000/29)**

(54) **EXTRAITS DE THE VERT CONTENANT DES CATECHOLS ET DE LA CAFEINE**

GRÜNTEE-EXTRAKTE ENTHALTEND CATECHOLE UND KOFFEIN

GREEN TEA EXTRACTS CONTAINING CATECHOLS AND CAFFEINE

(84) Etats contractants désignés:
**AT BE CH CY DE DK ES FI FR GB GR IE IT LI LU
MC NL PT SE**
Etats d'extension désignés:
**AL LT LV MK RO SI**

(30) Priorité: **14.01.1999 FR 9900328**

(43) Date de publication de la demande:
**16.05.2001 Bulletin 2001/20**

(73) Titulaire: **Laboratoires Arkopharma
06510 Carros (FR)**

(72) Inventeur: **ROMBI, Max
I-18022 Bordighera (IT)**

(74) Mandataire: **Ahner, Francis et al
Cabinet Régimbeau
20, rue de Chazelles
75847 Paris cedex 17 (FR)**

(56) Documents cités:
**EP-A- 0 456 023**

• **PATENT ABSTRACTS OF JAPAN vol. 009, no.
260 (C-309), 17 octobre 1985 (1985-10-17) &
JP 60 114153 A (OOSAKA YAKUHIN
KENKYUSHO:KK), 20 juin 1985 (1985-06-20)**
• **ADRIAN G. PASCHKA ET AL.: "INDUCTION OF
APOPTOSIS IN PROSTATE CANCER CELL
LINES BY THE GREEN TEA COMPONENT,
(-)-EPIGALLOCATECHIN-3-GALLATE."
CANCER LETTERS, vol. 130, no. 1-2, 14 août
1998 (1998-08-14), pages 1-7, XP002117137 NEW
YORK, NY, US ISSN: 0304-3835**

**Description**

La présente invention concerne le domaine général du traitement de l'obésité. Elle vise en particulier des compositions pour le traitement curatif et prophylactique de l'obésité, mais elle se rapporte également à la perte ou au maintien de poids d'un être humain pour améliorer sa silhouette.

L'objectif thérapeutique en matière d'obésité est bien défini: il s'agit soit de permettre au sujet de perdre du poids de façon significative, soit d'aider le sujet à conserver un niveau de poids aussi bas que souhaitable.

Plusieurs types d'approches ont été envisagés à ce jour.

Les approches nutritionnelles visent à réduire l'apport d'énergie sous forme d'aliments. Cela peut se faire en réduisant drastiquement les apports énergétiques ou en remplaçant des nutriments énergétiques par d'autres qui le sont moins: tels que les graisses non digestibles de substitution, les triglycérides structurés à assimilation réduite ou les fibres alimentaires non assimilables.

Les approches thérapeutiques peuvent avoir des cibles diverses.

- La réduction de la prise alimentaire peut être le premier objectif. La réduction de la prise alimentaire peut être recherchée par l'utilisation de substances anorexigènes, dont les effets à court terme sont montrés, mais dont la durée d'utilisation est limitée à cause d'effets secondaires indésirables. En fait, très peu de ces produits sont véritablement utilisables et leur efficacité à long terme reste très discutée. De nouvelles molécules sont en cours d'évaluation ou pourraient l'être dans un avenir proche, mais leur intérêt reste encore à démontrer.
- Un deuxième objectif peut être l'augmentation de la dépense énergétique par l'utilisation des substances thermogéniques agissant au niveau central ou périphérique. L'utilisation de ces substances reste encore limitée.
- Un troisième objectif est de réduire l'assimilation des lipides alimentaires, voire éventuellement celle des glucides. Il s'agit d'une approche plus récente mais qui connaît un intérêt grandissant. Une réduction de l'assimilation des lipides alimentaires peut être obtenue soit par une réduction de l'activité des enzymes digestives concernées, soit en modifiant les propriétés des interfaces transportant les molécules lipidiques, émulsions, vésicules ou micelles.

L'usage traditionnel du thé est sous forme de tisane, pour laquelle on utilise l'extrémité des tiges, comportant les deux dernières feuilles et le bourgeon. Après la récolte, ces feuilles peuvent être soumises à une fermentation, entraînant une transformation des substances chimiques qu'elles renferment et notamment des catéchols, ce qui correspond au thé noir ou bien séchées immédiatement donnant ainsi le thé vert.

Outre les catéchols, le thé renferme de la caféine dont l'effet diurétique est bien connu. Cet effet diurétique est à l'origine de l'usage traditionnel du thé vert comme plante médicinale pour favoriser l'élimination rénale de l'eau, que ce soit dans les troubles urinaires ou en complément de régimes amaigrissants. La présence de caféine est également à l'origine de l'usage traditionnel du thé dans les états de fatigue (asthénie).

De nombreuses études épidémiologiques réalisées sur certaines populations ont clairement démontré les effets bénéfiques de l'ingestion chronique du thé, et plus particulièrement du thé vert. Ainsi, la consommation de thé vert à long terme serait anti-athérogène du fait de ses effets hypocholestérolémiants (Muramatsu et al. 1986, Yang et al. 1997) et sa capacité à prévenir l'oxydation des LDL dans la circulation (Tijburg et al. 1997). Le thé vert est également reconnu pour ces effets anti-mutagène et anti-cancérigène. Ainsi, il a été montré que le thé vert réduisait de manière significative le risque de cancers colorectaux, de la peau et du sein (Blot et al. 1997, Conney et al. 1997, Dreosti et al. 1997, Jankun et al. 1997, Ji et al. 1997).

L'usage traditionnel du thé vert comme diurétique se fait actuellement sous forme de tisanes, d'extrait liquides, de poudre de plantes ou d'extraits en gélules ou comprimés. Dans ces différentes formes, le thé vert, souvent associé à une autre plante diurétique, est généralement utilisé à une dose correspondant à 1 à 3 g de plante par jour.

Dans le cadre du screening de propriétés pharmacologiques de différentes plantes, il a été découvert que des extraits de thé vert avaient des propriétés remarquables permettant de les utiliser dans le traitement de l'obésité.

Le corps humain dépense en permanence de l'énergie pour son fonctionnement. Cette dépense énergétique a trois origines: le métabolisme, le travail musculaire et la thermogenèse qui correspond à l'énergie dépensée par l'organisme pour maintenir une température constante.

La dépense énergétique est compensée par l'énergie apportée par l'assimilation des aliments. Si l'apport énergétique de la ration alimentaire est strictement identique à la dépense énergétique, l'individu conserve un poids stable. Si l'apport énergétique est excédentaire, l'organisme stocke cette énergie sous forme de graisses (augmentation du poids) et s'il est déficitaire, l'organisme puise l'énergie qui lui manque en brûlant les graisses stockées (perte de poids). Toutefois, dans cette dernière situation de déficit énergétique rencontrée au cours des régimes amaigrissants, l'organisme va réagir pour économiser de l'énergie et réduire la thermogenèse. C'est le mécanisme de contrôle qui explique l'échec des régimes amaigrissants. En effet, après quelques semaines de perte de poids, les individus voient leur poids se stabiliser. S'ils veulent continuer à maigrir, ils doivent réduire encore leur alimentation.

On comprend donc tout l'intérêt de pouvoir augmenter de façon permanente la thermogenèse, notamment en période de régime hypocalorique où elle est abaissée. Différentes substances chimiques stimulent la thermogenèse, telles que la nicotine, l'éphédrine, l'aspirine, la caféine, etc., mais aucune d'entre elles n'a permis de réaliser un médicament pour le traitement de l'obésité dans la mesure où les doses nécessaires pour obtenir une augmentation de la thermogenèse entraînaient des effets secondaires importants, incompatibles avec un traitement nécessairement de longue durée, s'étendant généralement sur plusieurs mois.

Cet objectif a été atteint conformément à la présente invention grâce à un extrait de thé vert, Camellia sinensis, riche en catéchols.

La présente invention vise également l'utilisation d'un extrait selon les revendications 1 à 3 de thé vert doté de propriétés anti-lipase et/ ou thermogénique, pour la fabrication d'un médicament destiné au traitement curatif et prophylactique de l'obésité.

La présente invention s'étend enfin à l'utilisation d'un extrait de thé vert selon l'une quelconque des revendications 1 à 3 pour la fabrication d'une composition non médicamenteuse destinée à engendrer une perte de poids ou maintenir un niveau de poids aussi bas que souhaitable.

Dans le cadre de la présente invention, l'extrait de thé vert contient de 20 à 50%, en particulier de 20 à 30% en masse de catéchols exprimés en gallate d'épigallocatéchol (GEGC).

La teneur en catéchols, exprimée en gallate d'épigallocatéchol (GEGC), est par exemple avantageusement déterminée dans le cadre de la présente invention par la mise en oeuvre de la méthode analytique décrite ci-après.

On opère par chromatographie liquide.

Solution à examiner : A 0,200 g d'extrait, on ajoute 80 ml de méthanol R. On place sous agitation magnétique pendant 5 min., puis dans un bain à ultrasons pendant 5 min. On filtre sur papier et on complète le volume à 100 ml avec le même solvant. On dilue la solution au cinquième dans le méthanol R.

Solution mère de caféine : On dissout 30 mg de caféine dans du méthanol et on complète à 100 ml avec le même solvant.

Solution mère de gallate d'épigallocatéchol : On dissout 6 mg de gallate d'épigallocatéchol (GEGC) dans du méthanol et on complète à 10 ml avec le même solvant.

Solution témoin : On prélève 1 ml de chaque solution mère et on complète à 10 ml avec le même solvant.

La chromatographie peut être réalisée en utilisant :

- une colonne en acier inoxydable de 250 mm de longueur et d'un diamètre intérieur de 4,6 mm remplie de gel de silice octadécylsililé pour chromatographie R (5 μm) thermostatée à 20°C (Nucléosil C18) et d'une précolonne ayant les mêmes caractéristiques que la colonne,
- comme phase mobile, à un débit de 1 ml/min, un mélange d'une solution aqueuse d'acide acétique glacial à 2% V/V (A) et d'acétonitrile (B) dont le gradient d'élution linéaire est le suivant :

| Temps (min) | A (%) | B (%) |
|---|---|---|
| 0 | 95 | 5 |
| 5 | 95 | 5 |
| 10 | 90 | 10 |
| 17 | 85 | 15 |
| 30 | 82 | 18 |
| 35 | 82 | 18 |
| 40 | 95 | 5 |

- comme détecteur un spectrophotomètre réglé à 278 nm.

On injecte séparément, au moins 2 fois, 10 μl de chacune des solutions. On ajuste la sensibilité du détecteur de façon à obtenir des pics dont la hauteur représente 50% au minimum de l'échelle totale de l'enregistreur.

On calcule la teneur en caféine, pour cent, à l'aide de l'expression suivante :

$$\frac{SCE}{SCT} x \frac{MCT}{MCE} x 0,05$$

SCE : surface du pic correspondant à la caféine dans le chromatogramme obtenu avec la solution à examiner
SCT : surface du pic correspondant à la caféine dans le chromatogramme obtenu avec la solution témoin

MCE : prise d'essai d'extrait dans la solution à examiner, exprimée en grammes

MCT :prise d'essai de caféine dans la solution témoin, exprimée en milligrammes.

On calcule la teneur en catéchols exprimés en gallate d'épigallocatéchol (GEGC), pour cent, à l'aide de l'expression suivante :

$$\frac{\Sigma SE}{ST} x \frac{MT}{ME} x 0{,}5$$

$\Sigma$SE :somme des surfaces des pics (2-5-6-7-8) correspondant aux catéchols dans le chromatogramme obtenu avec la solution à examiner

ST : surface du pic correspondant au GEGC dans le chromatogramme obtenu avec la solution témoin

ME : prise d'essai d'extrait dans la solution à examiner, exprimée en grammes

MT : prise d'essai de GEGC dans la solution témoin, exprimée en milligrammes.

Il convient de préciser que le gallate d'épigallocatéchol représente en moyenne environ 70 % de l'ensemble des catéchols présents dans un extrait de thé vert, avec une fourchette comprise entre 50 et 90 %.

Selon une caractéristique particulière de la présente invention, l'extrait de thé vert contient de 5 à 10% en masse de caféine.

Selon une autre caractéristique de l'invention, l'extrait de thé vert présente un rapport de la concentration en catéchols à la concentration en caféine compris entre 2 et 10.

Selon une caractéristique préférentielle de l'invention, l'extrait de thé vert est titré de manière à permettre l'administration d'une dose journalière de 250 à 500 mg, de préférence d'environ 375 mg de catéchols par jour et de 50 à 200 mg, de préférence d'environ 150 mg de caféine par jour.

L'augmentation de la thermogenèse chez le rat par un extrait de thé vert selon l'invention a été étudiée selon le protocole suivant :

On mesure la consommation d'oxygène des rats, maintenus dans une enceinte hermétique pendant deux heures et plus, après l'administration du produit testé. La dépense énergétique étant proportionnelle à la consommation d'oxygène, cette technique permet de mesurer l'augmentation de la thermogenèse, le métabolisme de base et le travail musculaire étant constant avant et après traitement.

Le produit testé était un extrait de thé vert contenant 24,7% de catéchols et 8,35% de caféine.

Les résultats suivants ont été obtenus :

Témoins: 0,06 w/kg$^{0,75}$

0,5 mg d'extrait/kg: 0,45 w/kg$^{0,75}$

1,0 mg d'extrait/kg: 0,81 w/kg$^{0,75}$

2,0 mg d'extrait/kg: 1,10 w/kg$^{0,75}$

L'augmentation de la thermogenèse chez l'homme par un extrait de thé vert selon l'invention a également été déterminée.

Une étude similaire a été réalisée sur 10 volontaires recevant à chaque repas soit 500 mg d'un extrait de thé vert (correspondant à 125 mg de catéchols et à 50 mg de caféine), soit 50 mg de caféine, soit un placebo.

La dépense énergétique totale sur 24h montrait une augmentation statistiquement significative ($p < 0{,}01$) en faveur de l'extrait: 9.867 kJ contre 9.538 kJ pour le placebo et 9.599 kJ pour la caféine.

Ces résultats démontrent la capacité d'un extrait de thé vert selon l'invention à augmenter significativement la thermogenèse. Cette propriété n'est pas liée à la teneur en caféine de l'extrait, puisque l'administration de caféine seule, à la même dose que celle apportée par l'extrait de thé vert, n'augmente pas la thermogenèse.

De plus, le fait que la baisse significative du Quotient Respiratoire ne soit pas accompagnée d'une augmentation de l'excrétion urinaire d'azote, permet de conclure à une augmentation de l'oxydation des lipides, ce qui est le but recherché dans tout traitement de l'obésité.

Il a enfin pu être démontré que l'extrait de thé vert selon l'invention conduisait à une inhibition des lipases digestives. Une étude in vitro a en effet permis de démontrer que l'extrait de thé vert à la dose de 6 mg d'extrait pour 100 mg de lipides, permettait de supprimer partiellement l'émulsification des lipides, tant en milieu gastrique qu'intestinal. Lorsque l'on sait que l'émulsification des lipides est l'étape indispensable à l'action des lipases sur les lipides alimentaires, ces résultats peuvent expliquer la capacité d'inhibition des lipases digestives.

Une autre étude in vitro, réalisée dans des conditions reproduisant les conditions physiologiques (action successive sur la trioléine de la lipase gastrique puis de la lipase pancréatique) a démontré que l'extrait de thé vert, à la dose de 6mg/100mg de lipides, permettait une inhibition presque totale de la lipase gastrique (89% d'inhibition) et partielle de la lipase pancréatique (32% d'inhibition) soit une inhibition de la lipolyse totale de près de 40%.

Dans le cadre de la présente invention, des travaux in vitro ont été conduits pour démontrer l'existence d'une synergie entre le gallate d'épigallocatéchol et la caféine. Ces travaux ont été réalisés sur un modèle pharmacologique ex vivo de thermogenèse. Le principe est de mesurer la consommation d'oxygène d'un échantillon de tissu adipeux brun de rat ; la consommation d'oxygène est proportionnelle à la thermogenèse induite dans le tissu adipeux par les différentes substances testées.

Les résultats ci-dessous indiquent l'augmentation de consommation d'oxygène en fonction de la concentration en GEGC et/ou en caféine.

| Caféine | 100 µM | 0 | 0 | 100 µM | 100 µM |
|---|---|---|---|---|---|
| GEGC | 0 | 100 µM | 200 µM | 100 µM | 200 µM |
| Augmentation de consommation d'oxygène | Sans effet | Sans effet | + 40 % | Sans effet | + 140 % |

Ces résultats démontrent bien l'existence d'une synergie de stimulation de la thermogenèse pour une concentration de 200 µM de GEGC et 100 µM de caféine, soit un rapport CEGC / caféine de 2.

Compte tenu des autres effets pharmacologiques de la caféine (tachycardie, insomnie), il peut donc être souhaitable pour augmenter la thermogenèse de limiter la quantité de caféine et d'augmenter le rapport GEGC / caféine. C'est pour cette raison que, selon une variante avantageuse de l'invention, ce rapport sera de préférence compris entre 2 et 10.

Exemple d'obtention d'un extrait de thé vert

Le thé vert renferme en moyenne 6 à 7% de catéchols et 2 à 3% de caféine.

Afin d'obtenir les propriétés d'augmentation de la thermogenèse et d'inhibition des lipases digestives décrites ci-dessus, un apport suffisant en catéchols est nécessaire. Il est donc nécessaire de procéder à une extraction du thé vert permettant d'obtenir un extrait suffisamment concentré en catéchols.

A titre d'exemple, le procédé d'extraction suivant peut être mis en oeuvre: 1 kg d'extrémités de tiges, comportant les deux dernières feuilles et le bourgeon, de thé vert broyées sont extraites par percolation pendant 6 à 8h avec 10 kg d'éthanol à 80% (m/m). Après filtration, l'extrait est concentré sous vide partiel à une température maximale de 60°C. L'extrait concentré est ensuite séché par atomisation à 250°C maximum avec ou sans maltodextrine, en fonction des spécifications en traceurs retenues. Ce procédé permet d'obtenir un extrait contenant 20 à 30% de catéchols et 5 à 10% de caféine.

Cet exemple n'est pas limitatif et d'autres procédés d'extraction permettant d'obtenir un extrait suffisamment riche en catéchols peuvent être mis en oeuvre, notamment en faisant varier les proportions d'eau et d'éthanol, ou en ayant recours à d'autres solvants tels que l'eau, l'acétate d'éthyle, le méthanol, etc., seuls ou associés en eux. Le choix des solvants retenus permettra de faire varier les teneurs en catéchols et en caféine, l'objectif étant une teneur élevée en catéchols, puisque les catéchols sont principalement à l'origine des propriétés pharmacologiques démontrées ci-dessus.

Sans pour autant vouloir se limiter à une telle interprétation, il apparaît probable que le mécanisme d'activité des extraits de thé vert, objet de la présente invention, puisse s'expliquer comme suit. Les catéchols présents en forte concentration dans les extraits de thé vert selon l'invention exercent un effet inhibiteur sur la catéchol-O-méthyl-transférase (COMT), alors que la concentration en caféine des extraits de thé vert selon l'invention agit en inhibant les phosphodiestérases, ce qui conduit à une activité renforcée de la noradrénaline sur la thermogenèse.

**Revendications**

1. Extrait synergique thermogénique de thé vert contenant de 20 à 50 % en masse de catéchols exprimés en gallate d'épigallocatéchol (GEGC) et de 5 à 10 % en masse de caféine.

2. Extrait selon la revendication 1, **caractérisé en ce qu'**il contient de 20 à 30% en masse de catéchols exprimés en gallate d'épigallocatéchol (GEGC).

3. Extrait selon L'une des revendications 1 et 2, **caractérisé en ce qu'**il présente un rapport de la concentration en catéchols à la concentration en caféine compris entre 2 et 10.

**4.** Utilisation d'un extrait selon l'une quelconque des revendications 1 à 3 pour la fabrication d'un médicament destiné au traitement curatif et prophylactique de l'obésité.

**5.** Utilisation d'un extrait selon l'une quelconque des revendications 1 à 3 pour la fabrication d'une composition non médicamenteuse destinée à engendrer une perte de poids ou maintenir un niveau de poids aussi bas que souhaitable afin d'améliorer la silhouette.

**6.** Utilisation selon l'une des revendications 4 et 5, **caractérisé en ce que** l'extrait de thé vert est titré de manière à permettre l'administration d'une dose journalière de 250 à 500 mg, de préférence d'environ 375 mg de catéchols par jour et de 50 à 200 mg, de préférence d'environ 150 mg de caféine par jour.

**Claims**

**1.** Synergic thermogenic extract of green tea containing from 20% to 50% by mass of catechols expressed as epigallocatechol gallate (EGCG) and from 5% to 10% by mass of caffeine.

**2.** Extract according to Claim 1, **characterized in that** it contains from 20% to 30% by mass of catechols expressed as epigallocatechol gallate (EGCG).

**3.** Extract according to one of Claims 1 to 2, **characterized in that** it has a ratio of the concentration of catechols to the concentration of caffeine of between 2 and 10.

**4.** Use of an extract according to one of Claims 1 to 3 for the manufacture of a medicinal product which is intended for the curative and prophylactic treatment of obesity.

**5.** Use of an extract according to one of Claims 1 to 3 for the manufacture of a non medicinal product which is intended to bring about a loss of weight or to maintain a weight level which is as low as desired in order to enhance the figure.

**6.** Use according to either of Claims 4 and 5, **characterized in that** the extract of green tea is titrated so as to allow the administration of a daily dose of from 250 mg to 500 mg, preferably about 375 mg, of catechols per day, and from 50 mg to 200 mg, preferably about 150 mg, of caffeine per day.

**Patentansprüche**

**1.** Synergistischer, thermogener Extrakt von grünem Tee, der 20 bis 50 Gew.% Catechine, ausgedrückt als Epigallocatechingallat (EGCG), und 5 bis 10 Gew.% Coffein enthält.

**2.** Extrakt nach Anspruch 1, **dadurch gekennzeichnet, daß** er 20 bis 30 Gew.% Catechine, ausgedrückt als Epigallocatechingallat (EGCG), enthält.

**3.** Extrakt nach einem der Ansprüche 1 und 2, **dadurch gekennzeichnet, daß** er ein Verhältnis der Konzentration an Catechinen zur Konzentration an Coffein von 2 bis 10 aufweist.

**4.** Verwendung eines Extrakts nach einem der Ansprüche 1 bis 3 für die Herstellung eines Medikaments, das zur heilenden oder prophylaktischen Behandlung von Obesität bestimmt ist.

**5.** Verwendung eines Extrakts nach einem der Ansprüche 1 bis 3 für die Herstellung einer nicht-medizinischen Zusammensetzung, die dazu bestimmt ist, einen Gewichtsverlust auszulösen oder ein so niedrig wie erwünschtes Gewichtsniveau beizubehalten, um die Figur zu verbessern.

**6.** Verwendung nach einem der Ansprüche 4 und 5, **dadurch gekennzeichnet, daß** der Extrakt von grünem Tee auf solche Weise titriert ist, daß er die Verabreichung einer täglichen Dosis von 250 bis 500 mg, vorzugsweise etwa 375 mg Catechinen pro Tag und 50 bis 200 mg, vorzugsweise etwa 150 mg Coffein pro Tag gestattet.